# EUROPEAN PATENT SPECIFICATION

(11) **EP 4 171 722 B1**
(45) Date of publication and mention of the grant of the patent: **21.08.2024**
(21) Application number: 21743575.9
(22) Date of filing: 24.06.2021
(51) Int. Cl.: A61M 60/196, A61M 60/268, A61M 60/427, A61M 60/438, A61M 60/837, A61M 60/873

(54) **ARTIFICIAL HEART MUSCLE**
KÜNSTLICHER HERZMUSKEL
MUSCLE CARDIAQUE ARTIFICIEL

(30) Priority: 24.06.2020 IT 202000015208
(43) Date of publication of application: 03.05.2023
(73) Proprietor: Stichting Amsterdam UMC, 1081 HV Amsterdam (NL)
(72) Inventor: CIANCHETTI, Matteo, 06131 San Marco (Perugia) (IT); LORENZON, Lucrezia, 56020 S. Maria a Monte (PI) (IT); MASELLI, Martina, 56012 Calcinaia (PI) (IT); ZRINSCAK, Debora, 56025 Pontedera (IT); KLUIN, Jolanda, 3734VJ Den Dolder (NL)
(74) Representative: ABM Agenzia Brevetti & Marchi
(86) International application number: PCT/IB2021/055605
(87) International publication number: WO 2021/260614

(56) References cited:
- WO-A1-2011/004400
- WO-A2-2004/093731
- US-A- 4 863 461
- US-A1- 2010 191 036
- US-A1- 2016 310 652

## Description

### Field of the invention

The present invention relates to the field of biomedical devices that can be implanted on patients with cardiovascular diseases.

In particular, the invention relates to an artificial heart containing at least one artificial ventricle.

### Description of the prior art

As well known, heart failure is a chronic pathological condition in which the heart muscle is unable to pump enough blood into the cardiovascular circulation. This pathology can be related to one of the two ventricles (left or right), but in the worst cases it involves both heart ventricles. Furthermore, it is very likely that, whenever left heart failure occurs, it can also degenerate into right heart failure and vice versa. All these considerations highlight how heart failure is an extremely serious pathology that compromises the patient's survival and has a devastating effect on his quality of life.

It is worth noting that every year 26 million people around the world are affected by this disease. The reason behind this dramatic situation is that the only effective solution to this pathological condition is a heart transplant. However, before a patient can receive a donor heart, a large number of aspects need to be considered (e.g. compatibility between the donor and the patient such as blood type and medical condition of the donor). Additionally, many patients are ineligible for a heart transplant due to advanced age, coexistence with other conditions or other problems encountered during the extensive screening process. For this reason, to date 45% of patients on the waiting list for a heart transplant die while waiting for a compatible donor.

In this scenario, the importance of developing artificial medical devices capable of completely replacing the ventricular pumping function and, consequently, reducing the number of people waiting for a donor or who are not suitable for transplantation is evident.

To solve the technical problem presented, different solutions have been designed, depending on the severity of the patient's cardiac dysfunction. When the infarct level of myocardial tissue is not chronic, natural pumping function can be simply assisted by Ventricular Assist Devices (VAD) or Direct Cardiac Compression devices (DCC). In the worst cases, however, when the patient has a chronic disease (i.e. end-stage patients), the damaged ventricles must be completely replaced and Total Artificial Hearts (TAH) are used for this.

Below are some significant examples of devices present in the state of the art.

Document US20160346449 belongs to the category of DCC and describes a device designed to wrap the heart muscle for inducing contractions by means of flexible hydraulic actuators. The device does not include an artificial ventricle.

Document JP4144014 describes a device suitable for simulating a left ventricle in the contracture stage. The device includes helical veins that favor the compression of the ventricle, but does not include actuators or biocompatible materials that allow the connection to the human circulatory system, being designed for the purposes of a laboratory experiment.

Document US20100298932 describes a device for assisting the pumping of blood that can be used to help or replace one or both ventricles. The device includes a chamber that is only partially deformable and cylindrical in shape, its deformation is not aimed at pumping blood.

Document US20150250934 describes a device designed to replace the left side of the heart or the two ventricles, faithfully reproducing their geometry and the mechanism of cardiac contraction. The actuation can be both electric and pneumatic and is incorporated in the ventricular chamber wall.

However, so far, the total artificial hearts that have reached the physiologically required cardiac output threshold in severe cases are those based on traditional pumping principles and are typically made from rigid materials. The main disadvantage of this type of device is their poor biocompatibility and haemocompatibility, mainly addressable to a huge conformity discrepancy between the device and surrounding tissues and device-induced changes in blood fluid dynamics.

Therefore, at present, a device capable of permanently and effectively replacing the human heart is far from clinically available and the 5-year risk of mortality after first hospitalization for heart failure is greater than 75%.

In US4863461 an artificial ventricle for replacing the human heart is described.

### Summary of the invention

It is therefore a feature of the present invention to provide a deformable artificial ventricle which allows to obtain a cardiac output comparable to that achieved by rigid devices based on traditional pumping principles.

It is also a feature of the present invention to provide such an artificial ventricle which allows to vary the fraction of blood expelled, both in the design phase and after the transplant, to adapt to the different needs of the patient.

It is still a feature of the present invention to provide such an artificial ventricle which reduces the damage to the surrounding natural tissues even with respect to deformable devices of the prior art.

These and other objects are achieved by an artificial ventricle according to claims from 1 to 13.

According to another aspect of the present invention, an artificial heart muscle according to claims from 14 to 15 is claimed.

In particular, at least one sensor is provided to monitor the functioning of the artificial ventricle.

### Brief description of the drawings

Further characteristic and/or advantages of the present invention are more bright with the following description of some embodiments thereof, exemplifying but not limitative, with reference to the attached drawings in which:
- Fig. 1A shows a possible embodiment of the artificial ventricle comprising a single ventricular chamber and a single active layer with fluidic actuators arranged in a helical arrangement;
- Fig. 1B shows an embodiment of Fig. 1A also comprising an inlet and an outlet for blood circulation;
- Figs. 2A and 2B show two following steps of the formation of inner folds in the ventricular chamber when passing between the expanded configuration and the compressed configuration;
- Fig. 2C shows a cross sectional view of the ventricular chamber in the compressed configuration;
- Fig. 2D shows a top plan view of the ventricular chamber in the compressed configuration;
- Fig. 3A shows a possible embodiment of the ventricular chamber wherein preferential folding directions are provided;
- Fig. 3B shows a perspective view an embodiment of Fig. 3A;
- Fig. 3C shows a top plan view an embodiment of Fig. 3A;
- Fig. 4A shows a possible embodiment of the artificial ventricle having fluidic actuators arranged in a circumferential arrangement and inner folds arranged in a longitudinal arrangement;
- Fig. 4B shows a possible embodiment of the artificial ventricle having fluidic actuators arranged in a longitudinal arrangement and inner folds arranged in a circumferential arrangement;
- Fig. 4C shows a possible embodiment of the artificial ventricle having fluidic actuators arranged in a double helix arrangement;
- Figs. 5A and 5B show, respectively in the expanded configuration and in the compressed configuration, a possible embodiment of the artificial ventricle wherein connection points are provided between the ventricular chamber and the fluidic actuators;
- Figs. 6A and 6B show, respectively in the expanded configuration and in the compressed configuration, a variant of the embodiment of the artificial ventricle of Figs. 5A and 5B wherein two active layers are provided;
- Fig. 7 shows a possible embodiment of the artificial ventricle wherein a matrix substance is provided;
- Figs. 8A and 8B show, respectively in the inactive state and in the active state, a possible embodiment of a fluidic actuator;
- Figs. 9A and 9B show, respectively in the active state and in the inactive state, a possible embodiment of a fluidic actuator;
- Fig. 10 shows a possible embodiment of the artificial ventricle comprising two separate ventricular chambers;
- Fig. 11 shows a possible embodiment of the artificial ventricle comprising two sub-chambers;
- Fig. 12 shows a possible embodiment of the artificial heart muscle comprising two ventricles.

### Description of some preferred embodiment

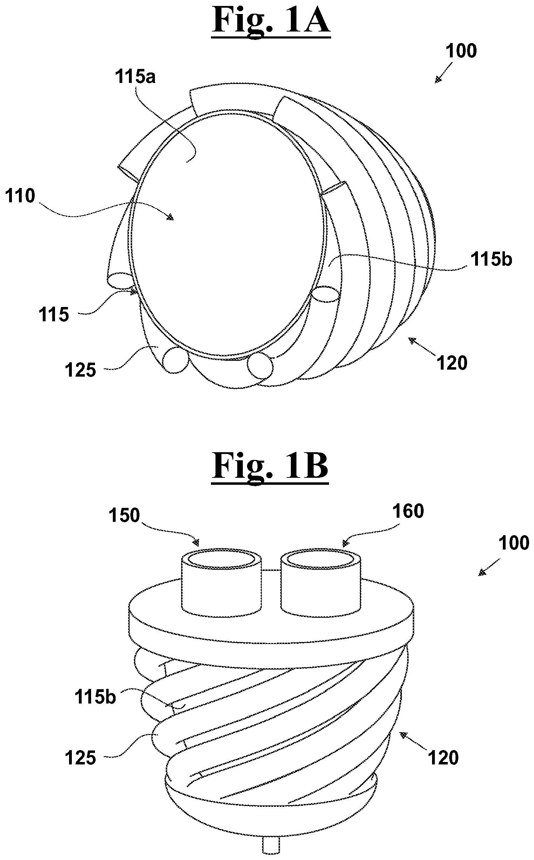

In Fig. 1A a first embodiment is shown of the present invention, wherein the artificial ventricle 100 comprises a containment wall 115 having an inner surface 115a and an outer surface 115b, defining an inner volume *V*.

The artificial ventricle 100 also comprises an active layer 120 consisting of a plurality of fluidic actuators 125 arranged to externally wind the ventricular chamber 110. In particular, each fluidic actuator 125 is adapted to exert a variable pressure ***Pᵥₐᵣ*** on the containment wall 115 to cyclically pass the ventricular chamber 110 between a compressed configuration wherein there is ***V*** = ***V_{c}*** and an expanded configuration wherein there is ***V* = *Vₑ* > *V_{c}.***

In Fig. 1B is shown the embodiment of Fig. 1A also comprising an inlet 150 and an outlet 160 for blood circulation. In particular, the inlet 150 is arranged to be connected by a valve to the right or left atrium of the patient in order to allow blood to enter the ventricular chamber 110, whereas the outlet 160 is arranged to be connected by a valve to the pulmonary artery or aorta of the patient to release blood from the ventricular chamber 110.

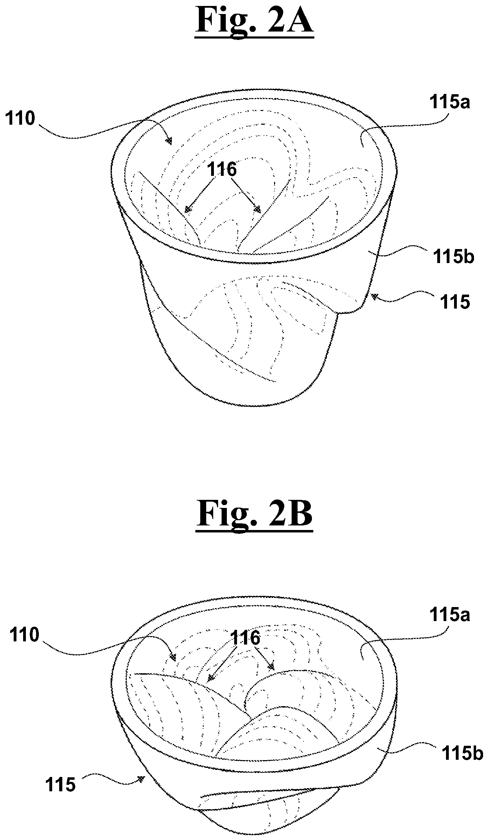

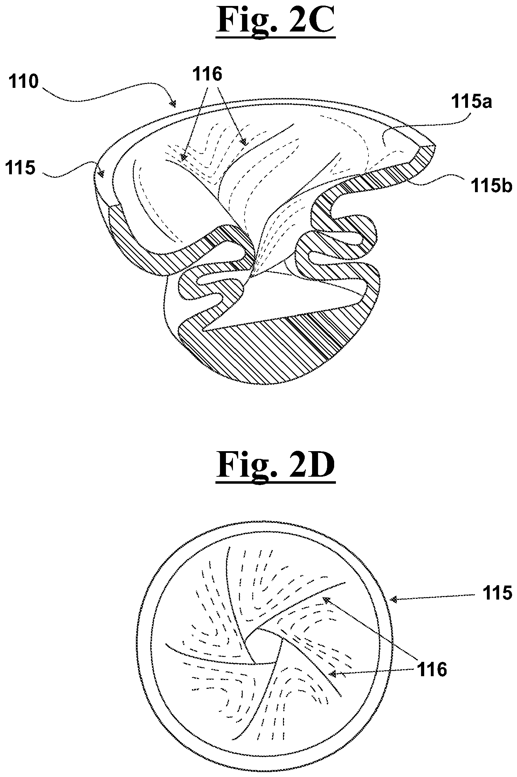

In particular, with reference to Figs. 2A, 2B, 2C and 2D, the containment wall 115 that defines the ventricular chamber 110 is made of deformable material, in such a way that, when passing from the expanded configuration to the compressed configuration, the containment wall 115 forms and/or increases the inner folds 116 that allow to reduce the inner volume ***V*** from the initial value ***Vₑ*** to the final value ***V_{c}*.**

Compared to the deformable artificial ventricles of the prior art, the generation of inner folds 116 makes it possible to obtain a higher efficiency during the reduction of the inner volume ***V*,** i.e. a higher variation of volume under a same external pressure ***Pᵥₐᵣ*** exerted on the containment wall 115.

In this way, the present invention allows to obtain a cardiac output comparable to that achieved by rigid devices based on traditional pumping principles but, with respect to the latter, thanks to the completely deformable material of the containment wall 115, it allows to greatly reduce the possibility of damaging the surrounding tissues, both during the implantation of the ventricle and during its operation.

Furthermore, depending on the specific shape of the ventricular chamber 110 and of the active layers 120, it is possible to vary, both during construction and in use, the fraction of blood expelled and the deformation geometry, adapting the artificial ventricle 100 to the specific needs of the patient.

In particular, the active layers 120 are made of deformable material, for example of elastomeric or polymeric material. This allows the fluidic actuators 125 to completely adhere to the containment wall 115, further reducing the possibility of damaging the surrounding tissues.

In particular, the inner folds 116 can be present also in the expanded configuration and increase during the compression.

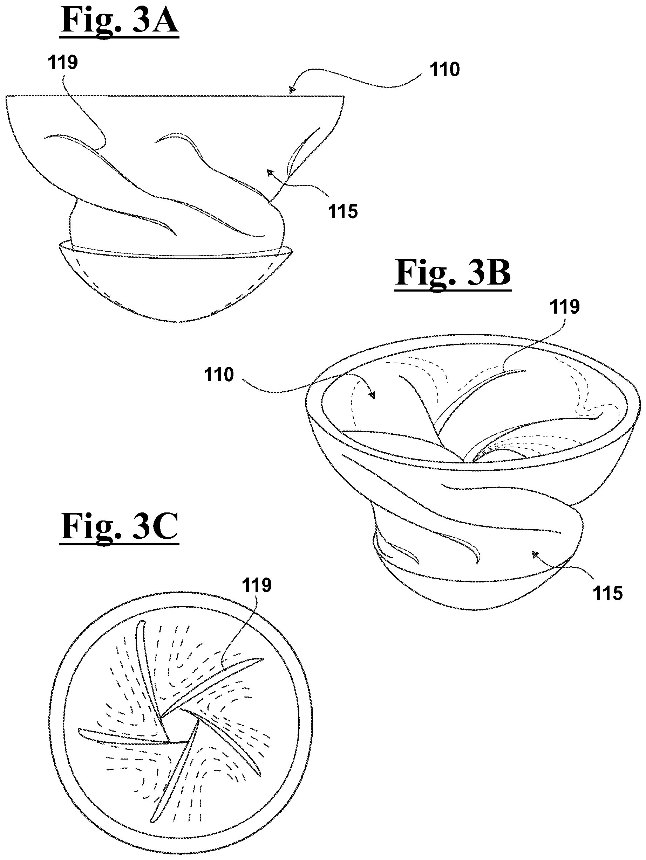

With reference to Figs. 3A, 3B and 3C, in a possible embodiment of the present invention, preferential folding directions 119 can be defined on the containment wall 115 in correspondence with which the formation of and/or the increase of the inner folds 116 is favored when passing from the expanded configuration to the compressed configuration.

For example, the preferential folding directions 119 can be arranged on the containment wall 115, alternatively, or in combination, according to:
- a longitudinal arrangement;
- a circumferential arrangement;
- a helical arrangement.

According to the way in which the folding directions are arranged, the type of deformation of the ventricular chamber can be varied, consequently varying the fraction of blood expelled at each cycle.

In a possible embodiment, the preferential folding directions 119 are made by previously folding the containment wall 115 to generate tensions arranged to guide the formation of the inner folds 116.

In a possible embodiment, the containment wall 115 comprises stiffeners in correspondence with the preferential folding directions 119.

In particular, these stiffeners are thickenings of the containment wall.

Alternatively, the stiffeners are elements made of a material other than the containment wall.

In particular, stiffeners are fibres of deformable and inextensible material, e.g. cotton.

In a possible embodiment, the preferential folding directions 119 are achieved by weakening the containment wall 119.

In particular, in correspondence with the preferential folding directions 119, the containment wall has a lower thickness.

In particular, the ventricular chamber 110, in the expanded configuration, has a shape selected from the group consisting of:
- a semi-ellipsoidal shape;
- an ellipsoidal shape;
- a paraboidal shape;
- a spherical shape;
- a combining the previous.

In particular, the inner surface 115a is made of biocompatible and hemocompatible material, allowing direct contact between the blood and the internal surface 115a of the containment wall 115.

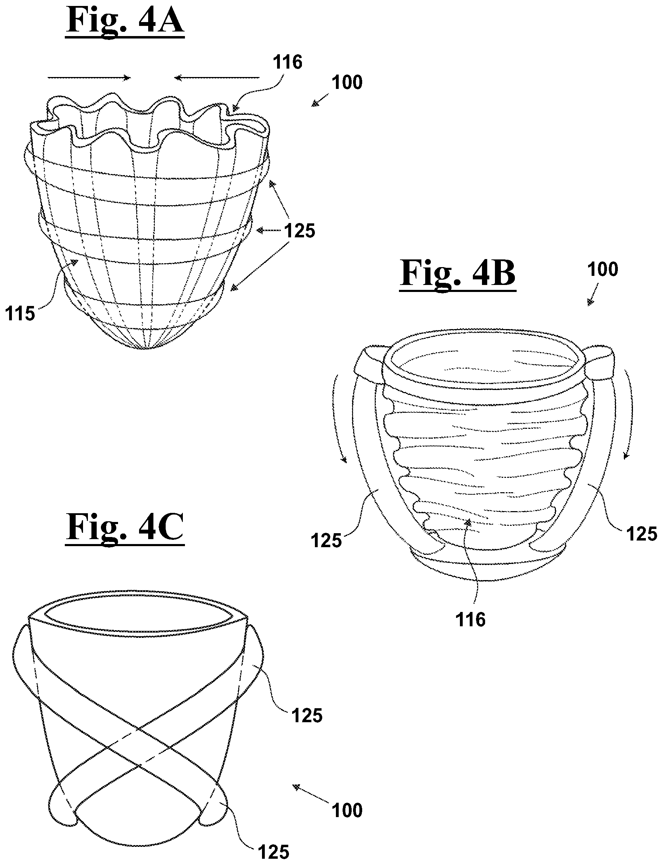

With reference to Figs. 4A, 4B and 4C, the fluidic actuators 125 can be arranged according to different geometries. Depending on the way in which the fluidic actuators 125 are arranged, it is possible to modify the type of pressure exerted and to have preferential deformation directions of the ventricular chamber 110.

For example, Fig. 4A shows an embodiment of the artificial ventricle 100 having fluidic actuators 125 arranged in a circumferential arrangement and inner folds 116 arranged in a longitudinal arrangement, whereas Fig. 4B shows an embodiment of the artificial ventricle 100 having fluidic actuators 125 arranged in a longitudinal arrangement and inner folds 116 arranged in a circumferential arrangement. Finally, Fig. 4C shows a possible embodiment of the artificial ventricle having fluidic actuators 125 arranged in a double helix arrangement.

Furthermore, the fluidic actuators 125 can be actuated simultaneously or alternately, and each fluidic actuator 125 can exert a pressure of adjustable intensity. In this way, the individual actuators of the same active layer can be actuated in a different way, for example by actuating only half actuators of a layer 120, to vary the quantity of blood expelled over time, by means of a volumetric ratio variation and/or compression speed variation.

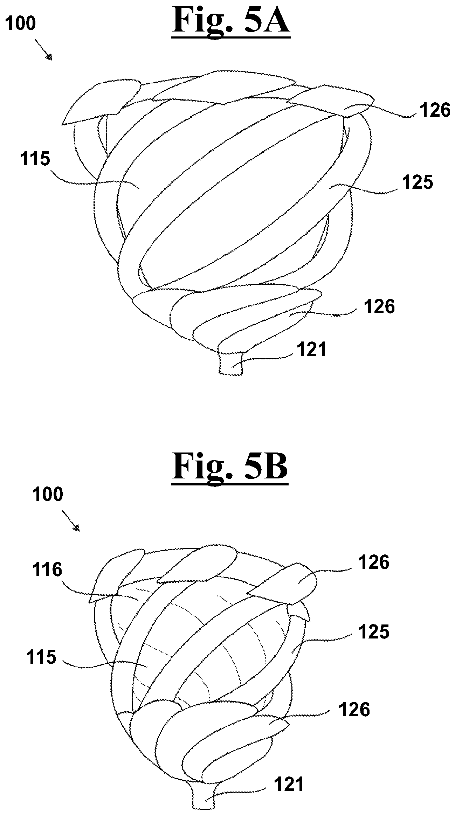

With reference to Figs. 5A and 5B, in an embodiment of the present invention, the fluidic actuators 125 are connected to the ventricular chamber 110 at least at one connection point 126, in particular at the basis and at the top of the containment wall 115. The connection between the fluidic actuators 125 and the ventricular chamber 110 allows transmitting the variable pressure ***Pᵥₐᵣ*** at predefined points, allowing the deformation of the ventricular chamber 110 and the formation of internal folds 116 to be managed more accurately.

Furthermore, the fluidic actuators 125 can be pneumatic actuators fed through an air inlet 121.

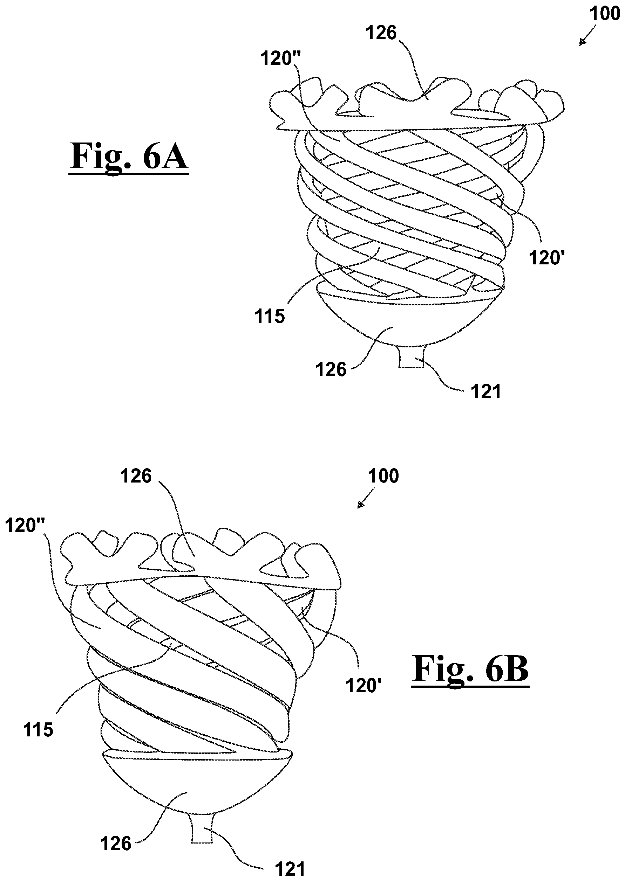

With reference to Figs. 6A and 6B, in a possible embodiment of the present invention, two active layers 120',120" ' are provided that can be simultaneously or alternately operated. This allows adjusting the action of the compression exerted on the ventricular chamber 110 changing the activity of the individual active layers 120. In this way, even once it has been made and implanted in the ventricle, it is still possible to adapt it to the evolution of the patient's medical conditions.

It is also possible to realize the active layers with actuators arranged according to different geometries. If, for example, a first active layer 120 comprises actuators arranged in a helical manner, while a second layer comprises actuators arranged in a longitudinal manner, it is possible to balance the action exerted by each layer to obtain a desired pressure and direction of deformation on the ventricular chamber.

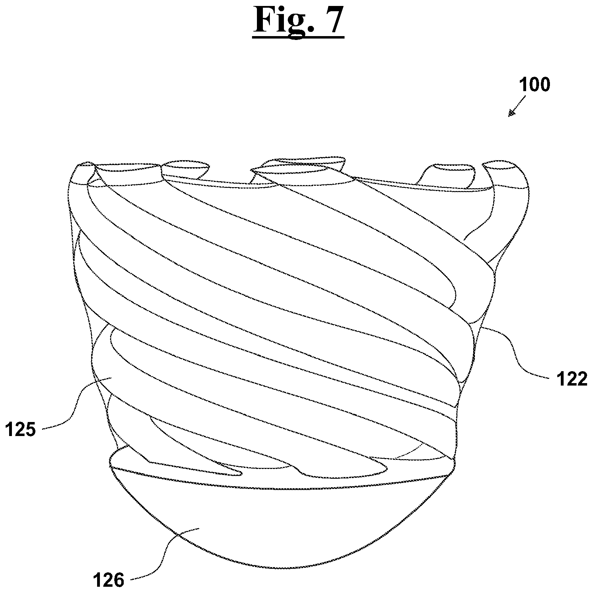

With reference to Fig. 7, in a possible embodiment of the artificial ventricle 100 a matrix substance 122 is also provided in which the active layer 120 or the active layers 120',120" ' are immersed. The matrix substance 122 allows both to constrain the relative movements between actuators of the same active layer and/or different active layers, and to have a more uniform action of the active layers on the ventricular chamber.

For example, the matrix substance may be a matrix foam or a thin elastomeric layer.

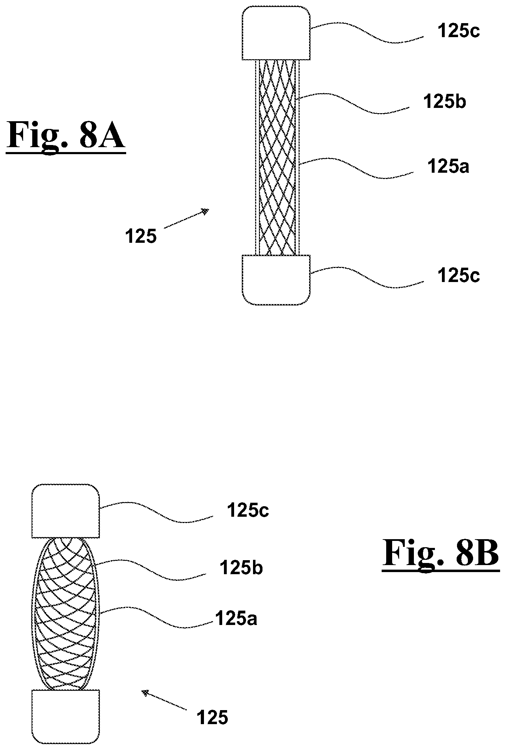

In particular, with reference to Figs. 8A and 8B, each fluidic actuator 125 can be an artificial pneumatic muscle of the "McKibben" type comprising an inflatable bladder 125a able to expand and/or compress and a reinforcement structure 125b able to control the expansion of the inflatable bladder 125a.

In particular, the inflatable bladder 125a can be constrained by the reinforcement structure 125b to expand and compress longitudinally and/or radially.

In this way, at the moment of pressurization, the inflatable bladder 125a tends to expand, both in the axial and radial direction. However, the geometry of the woven mesh, combined with the inextensibility of the threads that compose it, facilitate a deformation with respect to the other, generating a radial expansion and an axial contraction, i.e. a transition from the state of Fig. 8A to that of Fig. 8B.

Referring, for example, to the embodiment of Figs. 6A and 6B, the artificial muscles 125 are wound helically around the ventricular chamber 110 in two layers with opposite winding directions. After pressurization, the muscles 125 shorten in length and expand radially by applying a simultaneous contractile action on both axes of the ventricular chamber 110, thus generating internal folds 116. When depressurized, the muscles 125 lengthen, returning to the expanded configuration. The spatial orientation of the actuators 125 is therefore functional to the type of folds 116 generated and therefore to the overall quantity of blood expelled. In particular, the presence of a double layer of actuators 125 allows the possibility of varying the type and direction of the folds 116 during activation, in order to adapt them to the patient's needs.

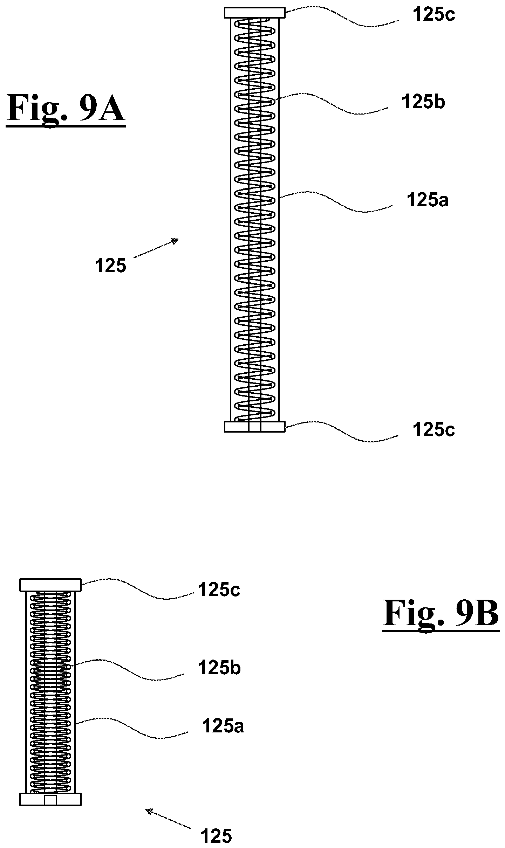

Alternatively, with reference to Figs. 9A and 9B, the fluidic actuators 125 can be artificial muscles of the "Inverse Pneumatic Artificial Muscle" (IPAM).

This type of actuator provides a cylindrical pneumatic chamber 125a and an external reinforcement structure 125b, cylindrical and coaxial to the pneumatic chamber. The external structure 125b has the function of guiding the deformation of the actuator, in response to the application of an internal pressure. The external structure 125b provides for the presence of one or more flexible and inextensible stiffening elements, or flexible and with a much higher stiffness than the material that constitutes the pneumatic chamber 125a. These elements can have the form of circular rings or cylindrical helices. The one or more stiffening elements can be mobile or fixed. The presence of one or more stiffening elements defines the axial direction of deformation as the preferred direction of the actuator. Radial deformation is prevented or severely limited, in relation to the stiffness of one or more stiffening elements. The external structure can be connected to the ends of the internal pneumatic chamber or along its entire length.

The two ends of the actuator can have end-fittings 125c for connection with the pressure source, pneumatic or fluidic, for the possible closure of one of the two ends and for fixing the actuator itself to the structure on which it exercises its own contractile action.

The operation of this type of actuator follows an inverse pattern with respect to the class of traditional artificial muscles, for which an increase in internal pressure determines a shortening of the actuator. This shortening is also usually accompanied by an increase in the radial dimensions of the actuator. On the other hand, IPAMs provide for an active elongation phase and a passive contraction phase. During the active phase, the internal pressure of the actuator is increased and it responds with an elongation along its main axis. In this phase, the material accumulates a certain amount of elastic energy that will be exploited during the passive phase of contraction, which begins in conjunction with a decrease in internal pressure.

The use of an inverse pattern of implementation of the artificial muscles is reflected in an inverse pattern of implementation of the entire artificial ventricle system. In particular, the ventricular chamber 110 is in its expanded volume configuration ***Vₑ*** when the artificial muscles are active and elongated. As a result of a decrease in pressure within the actuating layer, the artificial muscles wish to reach their resting length and contract. In this phase, they exert pressures on the ventricular chamber which induce the transition from its expanded form to its contracted form with volume ***V_{c}*.** The specific contractile characteristics of the artificial muscles of the actuating layer and their geometric arrangement around the ventricular chamber are functional to the definition of the geometry of the ventricular chamber in its contracted form, and therefore to the determination of the fraction of blood expelled during the contraction of the artificial ventricle.

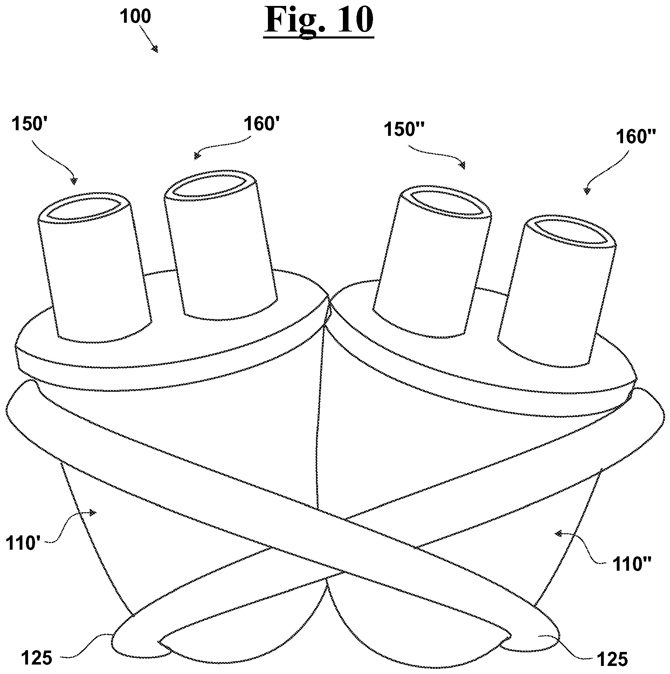

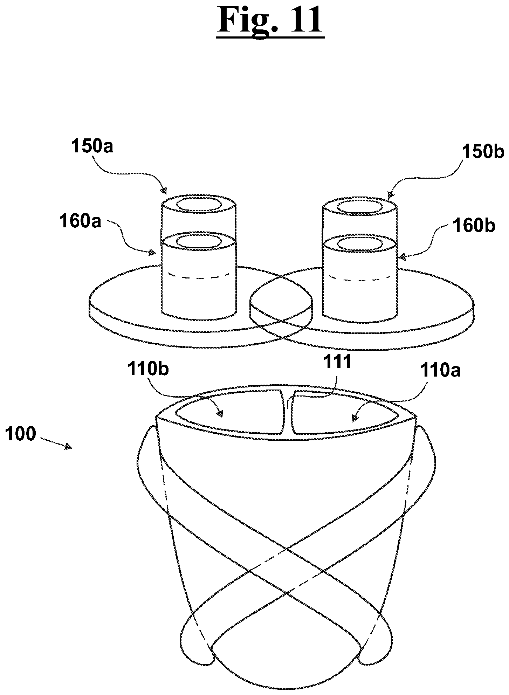

With reference to Figs. 10 and 11, in some embodiments provided by the present invention, the artificial ventricle 100 can be implanted to replace both the right and left ventricles.

In particular, in the embodiment of Fig. 10 the artificial ventricle 100 comprises two ventricular chambers 110',110'', each ventricular chamber 110',110" ' comprising an inlet 150',150'' and an outlet 160',160''. In this embodiment, the active layers 120 externally wind both the ventricular chambers 110',110'', compressing them simultaneously.

Alternatively, with reference to Fig. 11, in a further embodiment, the artificial ventricle 100 can comprise a single ventricular chamber 110, wherein a septum 111 is provided arranged to divide the ventricular chamber 110 in two sub-chambers 110a,110b, each sub-chamber 110a,110b comprising an inlet 150a,150b and an outlet 160a,160b.

In this embodiment, the active layers 120 externally wind both the sub-chambers 110a,110b, compressing them simultaneously.

In particular, the system can be designed to generate a higher pressure on a sub-chamber (e.g. by means of a different material of the sub-chambers or a particular arrangement of the actuators) in order to compensate for the pressure difference between right and left circulation.

Compared to the embodiment with two separate ventricular chambers, the embodiment with two sub-chambers entails first of all the advantage of requiring a smaller number of actuators for the same amount of ejected blood. Secondly, the overall volume required for the replacement of both ventricles is lower, facilitating transplantation, for example, on female patients.

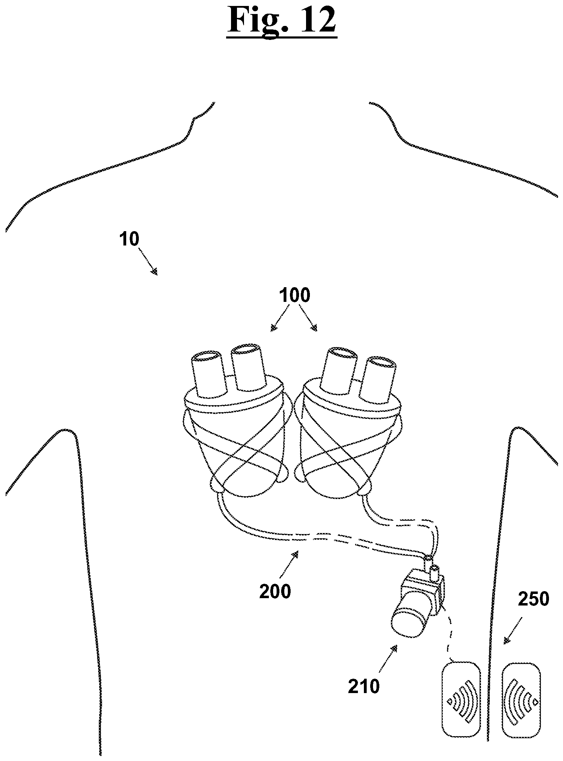

In Fig. 12 an artificial heart muscle 10 is shown, according to the present invention, comprising two artificial ventricles 100, a fluidic circuit 200 connected to each active layer 120 and a pump 210 connected to the fluidic circuit 200 to control the actuation of each active layer 120.

Advantageously, the artificial heart muscle 10 can comprise at least one sensor arranged to monitor the functioning of the artificial ventricle 100. In particular, the sensors acquire information on the pressure inside the ventricular chamber, the force exerted by the actuators and the operating status of the actuators (both in terms of damage and in terms of monitoring standard operation).

The artificial heart muscle 10 may further comprise a transcutaneous energy transfer system 250 adapted to provide electric energy to the pump 210 and, optionally, to the sensors from a source external to the patient's body. In particular, a wireless data transfer device can also be provided that allows to remotely control the activity of the pump and/or receive the data acquired by the sensors.

Alternatively to what is shown in Fig. 12, the artificial heart muscle 10, according to the present invention, can comprise artificial ventricles 100 of the type shown in Figs. 10 and 11.

In particular, in the case in which the artificial ventricle 100 comprises a ventricular chamber 110 divided into two sub-chambers 110a,110b or two ventricular chambers 110',110", the artificial heart muscle 10 comprises only an artificial ventricle 100 which replaces the biological ventricles left and right.

In particular, the inlet 150, 150a, 150b, 150', 150" of each chamber 110,110',110'' or sub-chamber 110a,110b is designed to be connected by means of a valve to the patient's right or left atrium to allow blood entering chamber 110,110',110'' or sub-chamber 110a,110b.

In particular, the outlet 160,160a,160b,16',160" of each chamber 110,110',110'' or sub-chamber 110a,110b is designed to be connected by means of a valve to the pulmonary artery or to the aorta of the patient for releasing blood from chamber 110,110',110'' or sub-chamber 110a,110b.

The foregoing description some exemplary specific embodiments will so fully reveal the invention according to the conceptual point of view, so that others, by applying current knowledge, will be able to modify and/or adapt in various applications the specific embodiments without further research and without parting from the invention, and, accordingly, it is meant that such adaptations and modifications will have to be considered as equivalent to the specific embodiments. The means and the materials to realise the different functions described herein could have a different nature without, for this reason, departing from the field of the invention.

## Claims

1. An artificial ventricle (100) comprising:
- at least one ventricular chamber (110) defined by a containment wall (115), said containment wall (115) having an inner surface (115a) and an outer surface (115b) and defining an inner volume ***V*;**
- at least one active layer (120) arranged to externally wind said ventricular chamber (110), said or each active layer (120) comprising at least one fluidic actuator (125) arranged to exert a variable pressure ***Pᵥₐᵣ*** on said containment wall (115) to cyclically pass said ventricular chamber (110) between a compressed configuration wherein there is ***V* = *V_{c}*** and an expanded configuration wherein there is ***V* = *Vₑ* > *V_{c};***
said artificial ventricle (100) **characterized in that** said containment wall (115) is made of deformable material,
**and in that,** when passing from said expanded configuration to said compressed configuration, said containment wall (115) is configured to form and/or to increase inner folds (116) for reducing said inner volume ***V*.**

2. The artificial ventricle (100), according to claim 1, wherein on said containment wall (115) preferential folding directions (119) are defined in correspondence of which the formation and/or the increase of said inner folds (116) is favoured when passing from said expanded configuration to said compressed configuration.

3. The artificial ventricle (100), according to claim 2, wherein said preferential folding directions (119) are arranged on said containment wall (115), alternatively, or in combination, according to:
- a longitudinal arrangement;
- a circumferential arrangement;
- a helical arrangement.

4. The artificial ventricle (100), according to claim 1, wherein said or each active layer (120) is made of deformable material.

5. The artificial ventricle (100), according to claim 1, wherein at least one fluidic actuator (125) is connected to said ventricular chamber (110) at least at one connection point (126).

6. The artificial ventricle (100), according to claim 1, wherein a matrix substance (122) is also provided in which said or each active layer (120) is immersed.

7. The artificial ventricle (100), according to claim 1, wherein said or each active layer (120) comprises at least two fluidic actuators (125) which can be simultaneously or alternately operated, each fluidic actuator (125) arranged to exert a pressure having adjustable intensity.

8. The artificial ventricle (100), according to claim 7, wherein said fluidic actuators (125) are arranged, alternatively, or in combination, according to:
- a longitudinal arrangement;
- a circumferential arrangement;
- a helical arrangement.

9. The artificial ventricle (100), according to claim 1, wherein at least two active layers (120',120'') are provided which can be simultaneously or alternately operated.

10. The artificial ventricle (100), according to claim 1, wherein said or each fluidic actuator (125) comprises:
- an inflatable bladder (125a) arranged to expand and/or to compress;
- a reinforcement structure (125b) arranged to control the expansion of said inflatable bladder (125a).

11. The artificial ventricle (100), according to claim 1, wherein only one ventricular chamber (110) is provided, said ventricular chamber (110) comprising an inlet (150) and an outlet (160).

12. The artificial ventricle (100), according to claim 1, wherein only one ventricular chamber (110) is provided and wherein a septum (111) is provided arranged to divide said ventricular chamber (110) in two sub-chambers (110a,110b), each sub-chamber (110a,110b) comprising an inlet (150a,150b) and an outlet (160a, 160b).

13. The artificial ventricle (100), according to claim 1, wherein two ventricular chambers (110',110") are provided, each ventricular chamber (110', 110") comprising an inlet (150',150") and an outlet (160',160").

14. An artificial heart muscle (10) arranged to be implanted in a patient for simulating the cardiac contraction, said artificial heart muscle (10) comprising:
- at least one artificial ventricle (100) according to any of claims from 1 to 13;
- at least one fluidic circuit (200) connected to each active layer (120).

15. The artificial heart muscle (10), according to claim 14, wherein are also provided:
- a pump (210) connected to said fluidic circuit (200) to control the actuation of each active layer (120);
- a transcutaneous energy transfer system (250) arranged to provide electric energy to said pump (210) .

## Patentansprüche

1. Künstlicher Ventrikel (100), umfassend:
- mindestens eine Ventrikelkammer (110), die durch eine Begrenzungswand (115) definiert ist, wobei die Begrenzungswand (115) eine innere Oberfläche (115a) und eine äußere Oberfläche (115b) aufweist und ein Innenvolumen ***V*** definiert;
- mindestens eine aktive Schicht (120), die dazu angeordnet ist, die Ventrikelkammer (110) extern zu umwickeln, wobei die oder jede aktive Schicht (120) mindestens einen Fluidaktor (125) umfasst, der dazu angeordnet ist, einen variablen Druck ***Pᵥₐᵣ*** auf die Begrenzungswand (115) auszuüben, um die Ventrikelkammer (110) zyklisch zwischen einer komprimierten Konfiguration, in der ***V* = *V_{c}*** gilt, und einer expandierten Konfiguration, in der ***V* = *Vₑ* > *V_{c}*** gilt, übergehen zu lassen;
wobei der künstliche Ventrikel (100) **dadurch gekennzeichnet ist, dass** die Begrenzungswand (115) aus verformbarem Material hergestellt ist
**und dass** die Begrenzungswand (115) beim Übergehen von der expandierten Konfiguration zu der komprimierten Konfiguration dazu konfiguriert ist, innere Falten (116) zu bilden und/oder zu vergrößern, um das Innenvolumen ***V* zu** reduzieren.

2. Künstlicher Ventrikel (100) gemäß Anspruch 1, wobei an der Begrenzungswand (115) bevorzugte Faltrichtungen (119) definiert sind, in Übereinstimmung mit denen die Bildung und/oder die Vergrößerung der inneren Falten (116) beim Übergehen von der expandierten Konfiguration zu der komprimierten Konfiguration begünstigt ist.

3. Künstlicher Ventrikel (100) gemäß Anspruch 2, wobei die bevorzugten Faltrichtungen (119) alternativ oder in Kombination gemäß Folgendem an der Begrenzungswand (115) angeordnet sind:
- einer Längsanordnung;
- einer umlaufenden Anordnung;
- einer spiralförmigen Anordnung.

4. Künstlicher Ventrikel (100) gemäß Anspruch 1, wobei die oder jede aktive Schicht (120) aus verformbarem Material hergestellt ist.

5. Künstlicher Ventrikel (100) gemäß Anspruch 1, wobei mindestens ein Fluidaktor (125) an mindestens einem Verbindungspunkt (126) mit der Ventrikelkammer (110) verbunden ist.

6. Künstlicher Ventrikel (100) gemäß Anspruch 1, wobei zudem eine Matrixsubstanz (122) bereitgestellt ist, worin die oder jede aktive Schicht (120) eingetaucht ist.

7. Künstlicher Ventrikel (100) gemäß Anspruch 1, wobei die oder jede aktive Schicht (120) mindestens zwei Fluidaktoren (125) umfasst, die gleichzeitig oder abwechselnd betrieben werden können, wobei jeder Fluidaktor (125) dazu angeordnet ist, einen Druck mit einstellbarer Intensität auszuüben.

8. Künstlicher Ventrikel (100) gemäß Anspruch 7, wobei die Fluidaktoren (125) alternativ oder in Kombination gemäß Folgendem angeordnet sind:
- einer Längsanordnung;
- einer umlaufenden Anordnung;
- einer spiralförmigen Anordnung.

9. Künstlicher Ventrikel (100) gemäß Anspruch 1, wobei mindestens zwei aktive Schichten (120', 120") bereitgestellt sind, die gleichzeitig oder abwechselnd betrieben werden können.

10. Künstlicher Ventrikel (100) gemäß Anspruch 1, wobei der oder jeder Fluidaktor (125) Folgendes umfasst:
- eine aufblasbare Blase (125a), die dazu angeordnet ist, zu expandieren und/oder zu komprimieren;
- eine Verstärkungsstruktur (125b), die dazu angeordnet ist, die Expansion der aufblasbaren Blase (125a) zu steuern.

11. Künstlicher Ventrikel (100) gemäß Anspruch 1, wobei nur eine Ventrikelkammer (110) bereitgestellt ist, wobei die Ventrikelkammer (110) einen Einlass (150) und einen Auslass (160) umfasst.

12. Künstlicher Ventrikel (100) gemäß Anspruch 1, wobei nur eine Ventrikelkammer (110) bereitgestellt ist und wobei ein Septum (111) bereitgestellt ist, das dazu angeordnet ist, die Ventrikelkammer (110) in zwei Unterkammern (110a, 110b) zu unterteilen, wobei jede Unterkammer (110a, 110b) einen Einlass (150a, 150b) und einen Auslass (160a, 160b) umfasst.

13. Künstlicher Ventrikel (100) gemäß Anspruch 1, wobei zwei Ventrikelkammern (110', 110") bereitgestellt sind, wobei jede Ventrikelkammer (110', 110") einen Einlass (150', 150") und einen Auslass (160', 160") umfasst.

14. Künstlicher Herzmuskel (10), der dazu angeordnet ist, in einen Patienten implantiert zu werden, um die Herzkontraktion zu simulieren, wobei der künstliche Herzmuskel (10) Folgendes umfasst:
- mindestens einen künstlichen Ventrikel (100) gemäß einem der Ansprüche 1 bis 13;
- mindestens einen Fluidkreislauf (200), der mit jeder aktiven Schicht (120) verbunden ist.

15. Künstlicher Herzmuskel (10) gemäß Anspruch 14, wobei zudem Folgende bereitgestellt sind:
- eine Pumpe (210), die mit dem Fluidkreislauf (200) verbunden ist, um die Betätigung jeder aktiven Schicht (120) zu steuern;
- ein transkutanes Energieübertragungssystem (250), das dazu angeordnet ist, der Pumpe (210) elektrische Energie bereitzustellen.

## Revendications

1. Ventricule artificiel (100) comprenant :
- au moins une chambre ventriculaire (110) définie par une paroi de confinement (115), ladite paroi de confinement (115) ayant une surface interne (115a) et une surface externe (115b) et définissant un volume interne ***V*,**
- au moins une couche active (120) agencée pour enrouler extérieurement ladite chambre ventriculaire (110), ladite ou chaque couche active (120) comprenant au moins un actionneur fluidique (125) agencé pour exercer une pression variable ***Pᵥₐᵣ*** sur ladite paroi de confinement (115) pour faire passer cycliquement ladite chambre ventriculaire (110) entre une configuration comprimée dans laquelle ***V* = *V_{c}*** existe et une configuration expansée dans laquelle ***V* = *Vₑ > V_{c}*** existe ;
ledit ventricule artificiel (100) **caractérisé en ce que** ladite paroi de confinement (115) est réalisée en matériau déformable,
**et en ce que,** lors du passage de ladite configuration expansée à ladite configuration comprimée, ladite paroi de confinement (115) est configurée pour former et/ou augmenter des plis internes (116) pour réduire ledit volume interne **V.**

2. Ventricule artificiel (100), selon la revendication 1, dans lequel sur ladite paroi de confinement (115) sont définies des directions de pliage préférentielles (119) en correspondance desquelles la formation et/ou l'augmentation desdits plis internes (116) est favorisée lors du passage de ladite configuration expansée à ladite configuration comprimée.

3. Ventricule artificiel (100) selon la revendication 2, dans lequel lesdites directions de pliage préférentielles (119) sont disposées sur ladite paroi de confinement (115), en alternance ou en combinaison, selon :
- une disposition longitudinale ;
- une disposition circonférentielle ;
- une disposition hélicoïdale.

4. Ventricule artificiel (100) selon la revendication 1, dans lequel ladite ou chaque couche active (120) est constituée d'un matériau déformable.

5. Ventricule artificiel (100) selon la revendication 1, dans lequel au moins un actionneur fluidique (125) est connecté à ladite chambre ventriculaire (110) au moins au niveau d'un point de connexion (126).

6. Ventricule artificiel (100) selon la revendication 1, dans lequel une substance matricielle (122) est également prévue dans laquelle ladite ou chaque couche active (120) est immergée.

7. Ventricule artificiel (100) selon la revendication 1, dans lequel ladite ou chaque couche active (120) comprend au moins deux actionneurs fluidiques (125) qui peuvent être actionnés simultanément ou alternativement, chaque actionneur fluidique (125) étant disposé pour exercer une pression ayant une intensité réglable.

8. Ventricule artificiel (100) selon la revendication 7, dans lequel lesdits actionneurs fluidiques (125) sont disposés, en alternance ou en combinaison, selon :
- une disposition longitudinale ;
- une disposition circonférentielle ;
- une disposition hélicoïdale.

9. Ventricule artificiel (100) selon la revendication 1, dans lequel au moins deux couches actives (120', 120") sont prévues qui peuvent être actionnées simultanément ou en alternance.

10. Ventricule artificiel (100), selon la revendication 1, dans lequel ledit ou chaque actionneur fluidique (125) comprend :
- une vessie gonflable (125a) disposée pour s'expanser et/ou se comprimer ;
- une structure de renfort (125b) disposée pour contrôler l'expansion de ladite vessie gonflable (125a).

11. Ventricule artificiel (100) selon la revendication 1, dans lequel une seule chambre ventriculaire (110) est prévue, ladite chambre ventriculaire (110) comprenant une entrée (150) et une sortie (160).

12. Ventricule artificiel (100) selon la revendication 1, dans lequel une seule chambre ventriculaire (110) est prévue et dans lequel un septum (111) est prévu agencé pour diviser ladite chambre ventriculaire (110) en deux sous-chambres (110a, 110b), chaque sous-chambre (110a, 110b) comprenant une entrée (150a, 150b) et une sortie (160a, 160b).

13. Ventricule artificiel (100), selon la revendication 1, dans lequel deux chambres ventriculaires (110', 110") sont prévues, chaque chambre ventriculaire (110', 110") comprenant une entrée (150', 150") et une sortie (160', 160").

14. Muscle cardiaque artificiel (10) conçu pour être implanté chez un patient pour simuler la contraction cardiaque, ledit muscle cardiaque artificiel (10) comprenant :
- au moins un ventricule artificiel (100) selon l'une quelconque des revendications 1 à 13 ;
- au moins un circuit fluidique (200) connecté à chaque couche active (120).

15. Muscle cardiaque artificiel (10), selon la revendication 14, dans lequel sont également prévus :
- une pompe (210) connectée audit circuit fluidique (200) pour commander l'actionnement de chaque couche active (120) ;
- un système de transfert d'énergie transcutané (250) conçu pour fournir de l'énergie électrique à ladite pompe (210).
